(19) Europäisches Patentamt
European Patent Office
Office européen des brevets

(11) Numéro de publication : **0 356 318 B1**

# (12) FASCICULE DE BREVET EUROPEEN

(45) Date de publication du fascicule du brevet : **28.10.92 Bulletin 92/44**

(51) Int. Cl.$^5$ : **C07C 319/14**

(21) Numéro de dépôt : **89402293.8**

(22) Date de dépôt : **16.08.89**

## (54) Synthèse de polysulfures organiques.

(30) Priorité : **23.08.88 FR 8811131**

(43) Date de publication de la demande : **28.02.90 Bulletin 90/09**

(45) Mention de la délivrance du brevet : **28.10.92 Bulletin 92/44**

(84) Etats contractants désignés : **AT BE CH DE ES FR GB GR IT LI LU NL SE**

(56) Documents cités :
**FR-A- 1 358 398**
**FR-A- 2 607 496**

(73) Titulaire : **SOCIETE NATIONALE ELF AQUITAINE (PRODUCTION)**
**Tour Elf, 2, Place de la Coupole, La Défense 6**
**F-92400 Courbevoie (FR)**

(72) Inventeur : **Vallee, Yannick**
**23 Chemin du Clos-Beaumois**
**F-14000 Caen (FR)**
Inventeur : **Labat, Yves**
**35 bis Rue Michel Houreau**
**F-64000 Pau (FR)**

(74) Mandataire : **Leboulenger, Jean et al**
**ATOCHEM Département Propriété Industrielle**
**F-92091 Paris la Défense 10 Cédex 42 (FR)**

EP 0 356 318 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne la fabrication de polysulfures organiques du type $RS_nR$ où les deux symboles R, identiques ou différents, représentent chacun un radical hydrocarboné et n est un nombre allant de 3 à 8, en particulier de 4 à 6.

Ces polysulfures qui ont trouvé diverses applications industrielles, notamment comme additifs extrême pression et comme agents de sulfuration de catalyseurs, sont actuellement préparés industriellement par réaction d'un mercaptan avec du soufre liquide en présence d'un catalyseur basique. Malgré une température assez élevée (130-140°C), la réaction :

$$2RSH + (n-1)S \rightarrow RS_nR + H_2S$$

est souvent incomplète. Les produits obtenus sont troubles et contiennent du mercaptan et du soufre libre ; ils comprennent en outre une proportion importante de polysulfures lourds en $S_6$, $S_7$, $S_8$ ... qui sont instables et déposent régulièrement du soufre.

L'emploi, comme catalyseur, de la combinaison d'un mercaptan avec un oxyde d'alcène et une base alcaline (brevet FR 2 607 496) conduit à une réaction pratiquement complète, mais alourdit sensiblement le coût de fabrication.

Il a maintenant été trouvé que l'utilisation de disulfure de carbone permet d'effectuer la réaction dans des conditions douces (10-20°C) tout en gardant des durées réactionnelles relativement courtes (quelques heures) et d'obtenir des polysulfures jaunes et limpides dont le taux de soufre particulièrement élevé peut atteindre la valeur moyenne de 5 atomes de soufre par molécule de polysulfure. La teneur en mercaptan (SH) des polysulfures ainsi obtenus est nulle (en tout cas inférieure à 5 ppm), ce qui indique une réaction complète. Malgré leur taux de soufre élevé, les produits obtenus contiennent peu de polysulfures lourds.

La combinaison du disulfure de carbone avec le mercaptide RSM formé par un mercaptan et une base conduit à la formation d'un thioxanthate

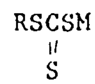

qui joue le rôle de catalyseur dans la production des polysulfures.

La présente invention a donc pour objet un procédé de préparation de polysulfures organiques par action du soufre sur un mercaptan, caractérisé en ce que la réaction est effectuée en présence d'un thioxanthate formé par combinaison du disulfure de carbone avec un mercaptide.

Bien que le thioxanthate puisse être préparé séparément en mélangeant des quantités sensiblement équimolaires d'un mercaptan, d'une base et de disulfure de carbone au sein d'un solvant organique inerte (par exemple le méthanol) et isolé par évaporation du solvant, le meilleur mode de réalisation du procédé selon l'invention consiste à former le thioxanthate in situ en agitant pendant quelques minutes un mélange de mercaptan, de base et de disulfure de carbone avant d'introduire le soufre nécessaire à la formation du polysulfure désiré.

La quantité de disulfure de carbone à utiliser peut varier dans de larges limites, pourvu qu'elle soit molairement au moins équivalente à celle de la base mise en oeuvre. Il est cependant avantageux d'opérer avec un excès de disulfure de carbone par rapport à la base et, plus particulièrement, en utilisant un volume de disulfure de carbone compris entre 0,5 et 1,5 fois celui du mercaptan mis en oeuvre.

Bien qu'on préfère utiliser un mercaptan bien déterminé pour obtenir un polysulfure symétrique, on ne sortirait pas du cadre de la présente invention en utilisant un mélange de mercaptans. Dans le ou les mercaptans de départ, le radical hydrocarboné peut être aliphatique, cycloaliphatique ou aromatique. On préfère cependant les alkylmercaptans contenant de 2 à 15 atomes de carbone et, plus particulièrement, ceux à radical alkyle tertiaire.

Les quantités respectives de soufre et de mercaptan à mettre en oeuvre dépendent du polysulfure désiré, c'est-à-dire du nombre moyen d'atomes de soufre par molécule de polysulfure. D'une façon générale, le rapport : atomes-grammes de soufre/moles de mercaptan peut varier de 0,5 à 5. Il est de préférence compris entre 1,5 et 2,5 pour obtenir des polysulfures dont le nombre moyen d'atomes de soufre par molécule est de 4 à 5.

La base employée peut être de nature minérale ou organique. On peut notamment utiliser un hydroxyde métallique tel que, par exemple, l'hydroxyde de sodium ou de potassium, ou une amine, en particulier une trialkylamine comme, par exemple, la triéthylamine. La base peut être employée en faible quantité (par exemple de 0,01 à 2 mole pour 100 moles de mercaptan), mais aussi en quantité importante (par exemple un équivalent molaire par rapport au mercaptan).

Contrairement aux méthodes antérieures qui nécessitent un chauffage au-dessus de 100°C entraînant la dégradation d'une partie des polysulfures en disulfures, la réaction selon l'invention ne nécessite pas de chauffage et est donc réalisée de préférence à la température ambiante (10-20°C). Cependant, on ne sortirait pas du cadre de la présente invention en opérant à une température plus basse ou plus élevée, par exemple de 0 à 46°C ou même à une température supérieure en opérant sous pression dans un réacteur fermé.

Les différents polysulfures obtenus selon l'invention présentent une répartition caractéristique. En effet, on n'observe pas de monosulfure, ni de disulfure et généralement peu de trisulfure ; le produit majoritaire est le tétrasulfure accompagné d'un peu de sulfures plus lourds.

La proportion relativement faible de sulfures lourds (n > 5) malgré un nombre n moyen élevé permet aux polysulfures obtenus selon l'invention d'être stables, c'est-à-dire de ne pas déposer de soufre au stockage et de rester limpides et jaunes. Les polysulfures obtenus par d'autres méthodes contiennent beaucoup de sulfures lourds ; ils déposent facilement du soufre et deviennent troubles.

Les exemples suivants illustrent l'invention sans la limiter.

EXEMPLE 1

a) Préparation de l'éthylthioxanthate de triéthylammonium

On agite pendant 15 minutes 7,4 ml (0,1 mole) d'éthylmercaptan et 13,8 ml (0,1 mole) de triéthylamine dans 30 ml de méthanol, puis on ajoute 6 ml (0,1 mole) de disulfure de carbone. Une réaction exothermique a lieu et le mélange réactionnel devient orangé. On agite encore pendant 3 heures, puis on évapore le méthanol.

On obtient ainsi 9 g d'une pâte orange dont les spectres de RMN $^1H$ et $^{13}C$ indiquent qu'il s'agit de l'éthylthioxanthate de triéthylammonium

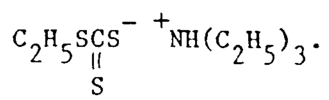

b) Synthèse du polysulfure de tertiobutyle

On agite pendant 2 heures à la température ambiante un mélange de 80 ml (0,71 mole) de tert-butylmercaptan, 80 ml de disulfure de carbone, 45,5 g (1,42 mole) de soufre et 0,1 g du thioxanthate obtenu ci-dessus. Après évaporation du disulfure de carbone, on obtient 93 g d'un liquide limpide jaune qui ne noircit pas le papier à l'acétate (absence de $H_2S$) et dont l'analyse indique que le nombre moyen d'atomes de soufre par molécule est de 5, correspondant au polysulfure de tertiobutyle de formule $(CH_3)_3CS_5C(CH_3)_3$.

EXEMPLE 2

On répète l'exemple 1-b, sauf qu'on opère en l'absence de disulfure de carbone et laisse sous agitation pendant 6 heures à la température ambiante. On obtient un polysulfure de formule moyenne $(CH_3)_3CS_{4,9}C(CH_3)_3$ sous forme d'un liquide limpide jaune ne noircissant pas le papier à l'acétate.

EXEMPLE 3

80 ml (1,08 mole) d'éthylmercaptan, 80 ml de disulfure de carbone et 0,4 ml (2,8 millimoles) de triéthylamine sont agités pendant une demi-heure à la température ambiante. On ajoute alors 69,12 g (2,16 moles) de soufre. Après 30 minutes d'agitation, tout le soufre a disparu. On agite encore pendant une heure et demie, puis on évapore le disulfure de carbone.

On obtient ainsi 111,5 g d'une huile jaune limpide qui ne noircit pas le papier à l'acétate (pas de $H_2S$). Son analyse RMN $^1H$ montre qu'il s'agit d'un mélange de polysulfures d'éthyle et son analyse élémentaire (S = 73,8 %) indique que le nombre moyen d'atomes de soufre par molécule est de 5.

EXEMPLE 4

On agite pendant 15 minutes à la température ambiante un mélange de 80 ml (0,861 mole) d'isopropylmercaptan, 80 ml de disulfure de carbone et 0,6 ml (4,3 millimoles) de triéthylamine. On ajoute ensuite 56,6 g (1,768 mole) de soufre solide et poursuit l'agitation pendant une heure.

Le disulfure de carbone et la triéthylamine sont alors évaporés par bullage d'azote et l'évaporation est achevée à l'évaporateur rotatif à 60°C sous 1600 Pa. On obtient ainsi 103 g d'un liquide jaune qui ne noircit pas le papier à l'acétate et dont le taux de mercaptan résiduel est inférieur à 5 ppm. Sa micro-analyse indique une teneur en soufre de 63,3 %, ce qui correspond à la formule moyenne :

$$(CH_3)_2CHS_{4,65}CH(CH_3)_2$$

Rendement : 97 %.

EXEMPLE 5

On opère comme à l'exemple 4 avec 80 ml (0,71 mole) de tert-butyl-mercaptan, 80 ml de disulfure de carbone, 0,8 ml (5,7 millimoles) de triéthylamine et 45,5 g (1,42 mole) de soufre. On obtient 92,3 g d'un liquide jaune limpide qui ne noircit pas le papier à l'acétate. L'analyse de son spectre RMN$^1$H indique la répartition molaire suivante :

| | |
|---|---|
| disulfure | :0 |
| trisulfure | : 1 % |
| tétrasulfure | : 48 % |
| pentasulfure | : 21 % |
| hexasulfure | : 12 % |
| hepta- et octasulfure | : 18 % |

Le nombre moyen d'atomes de soufre par molécule est de 4,9 et le rendement de 96 %.

EXEMPLE 6

Si on répète l'exemple 5, mais avec seulement 34,3 g (1,065 mole) de soufre, on obtient 76,5 g d'un polysulfure jaune limpide qui ne noircit pas le papier à l'acétate et dont le taux de mercaptan résiduel est inférieur à 5 ppm.

La microanalyse (S = 54,3 %) indique un nombre moyen de 4,3 atomes de soufre par molécule et l'analyse RMN$^1$H montre la répartition molaire suivante : $S_3$ = 1 %, $S_4$ = 69 %, $S_5$ = 19 %, $S_6$ = 7 %, $S_7$-$S_8$ = 4 %.

EXEMPLE 7

On opère comme à l'exemple 4 avec 80 ml (0,465 mole) de tert-octylmercaptan, 80 ml de disulfure de carbone, 0,4 ml de triéthylamine et 29,8 g (0,93 mole) de soufre.

On obtient 79 g d'un polysulfure jaune limpide qui ne noircit pas le papier à l'acétate et dont le taux de mercaptan résiduel est inférieur à 5 ppm. La microanalyse indique une teneur en soufre de 41,5 %, ce qui correspond à un nombre moyen de 5 atomes de soufre par molécule.

Rendement : 88 %.

EXEMPLE 8

On opère comme à l'exemple 4 avec 100 ml (0,425 mole) de tert-dodécylmercaptan, 100 ml de disulfure de carbone, 0,5 ml de triéthylamine et 27,2 g (0,85 mole) de soufre. La dissolution du soufre étant lente, on maintient l'agitation pendant 4 heures avant le bullage d'azote. L'évaporation à 60°C sous 1600 Pa doit être poursuivie pendant 5 heures pour que le liquide jaune limpide ne noircisse plus le papier à l'acétate que légèrement.

Le taux de mercaptan résiduel est inférieur à 5 ppm. La microanalyse indique une teneur en soufre de 31,7 %, soit un nombre moyen de 4,9 atomes de soufre par molécule.

EXEMPLE 9 (Comparatif à l'exemple 6)

On agite à température ambiante un mélange de 80 ml de tert-butylmercaptan, 0,8 ml de triéthylamine et 34,3 g de soufre, en l'absence de disulfure de carbone.

Après 16 heures d'agitation, on filtre le soufre solide qui n'a pas réagi. On obtient un polysulfure orangé trouble dont l'analyse RMN$^1$H indique la présence de trisulfure et donne la répartition molaire suivante : $S_3$ = 16 %, $S_4$ = 56 %, $S_5$ = 14 %, $S_6$ = 8 %, $S_7$-$S_8$ = 6 %.

Le nombre moyen en soufre est de 4 au lieu de 4,3 à l'exemple 6.

EXEMPLE 10

A une solution de 20 g (0,5 mole) d'hydroxyde de sodium dans 200 ml de méthanol, on ajoute 56 ml (0,5 mole) de tert-butylmercaptan et 40 ml de disulfure de carbone. On agite pendant 30 minutes, puis on ajoute 32 g de soufre et poursuit l'agitation pendant 16 heures.

On évapore ensuite le disulfure de carbone par strippage à l'air, puis on filtre le précipité ($Na_2S$) et évapore le méthanol à l'évaporateur rotatif. On obtient ainsi 46 g d'un polysulfure dont le nombre moyen d'atomes de soufre par molécule est de 4.

## Revendications

1. Procédé de préparation de polysulfures organiques par action du soufre sur un mercaptan, caractérisé en ce que la réaction est effectuée en présence d'un thioxanthate formé par combinaison du disulfure de carbone avec un mercaptide.

2. Procédé selon la revendication 1, dans lequel on opère à une température allant de 0 à 46°C, de préférence à la température ambiante.

3. Procédé selon la revendication 1 ou 2, dans lequel, par mole de mercaptan, on met en oeuvre de 0,5 à 5 atomes-grammes de soufre, de préférence de 1,5 à 2,5 atomes-grammes.

4. Procédé selon l'une des revendications 1 à 3 dans lequel le mercaptan est un alkylmercaptan et, de préférence, un alkylmercaptan tertiaire.

5. Procédé selon l'une des revendications 1 à 4, consistant à introduire du soufre dans un mélange d'un mercaptan, d'une base et de disulfure de carbone.

6. Procédé selon la revendication 5 dans lequel on utilise un volume de disulfure de carbone allant de 0,5 à 1,5 fois celui du mercaptan mis en oeuvre.

7. Procédé selon la revendication 5 ou 6, dans lequel la base employée est un hydroxyde métallique ou une amine, de préférence une trialkylamine.

8. Procédé selon l'une des revendications 5 à 7, dans lequel on utilise au moins 0,1 millimole de base par mole de mercaptan.

## Patentansprüche

1. Verfahren zur Herstellung von organischen Polysulfiden durch Einwirkung von Schwefel auf ein Mercaptan, dadurch gekennzeichnet, daß die Reaktion in Gegenwart eines durch die Kombination von Schwefelkohlenstoff mit einem Mercaptid gebildeten Thioxanthats durchgeführt wird.

2. Verfahren nach Anspruch 1, in dem man bei einer Temperatur von 0 bis 46°C, vorzugsweise bei Raumtemperatur, arbeitet.

3. Verfahren nach Anspruch 1 oder 2, in dem pro Mol Mercaptan 0,5 bis 5, vorzugsweise 1,5 bis 2,5 Grammatom Schwefel, eingesetzt werden.

4. Verfahren nach einem der Ansprüche 1 bis 3, in dem das Mercaptan ein Alkylmercaptan, vorzugsweise ein tertiäres Alkylmercaptan, ist.

5. Verfahren nach einem der Ansprüche 1 bis 4, in dem Schwefel einem Gemisch aus einem Mercaptan, einer Base und Schwefelkohlenstoff zugefügt wird.

6. Verfahren nach Anspruch 5, in dem man ein Volumen Schwefelkohlenstoff verwendet, das das 0,5 bis 1,5fache des eingesetzten Mercaptans beträgt.

7. Verfahren nach Anspruch 5 oder 6, in dem die verwendete Base ein Metallhydroxid oder ein Amin, vorzugsweise ein Trialkylamin, ist.

8. Verfahren nach einem der Ansprüche 5 bis 7, in dem man wenigstens 0,1 Millimol der Base pro Mol des Mercaptans verwendet.

**Claims**

1. Process for the preparation of organic polysulphides by reaction of sulphur with a mercaptan, characterised in that the reaction is performed in the presence of a thioxanthate formed by combination of carbon disulphide with a mercaptide.

2. Process according to Claim 1, in which the operation is carried out at a temperature ranging from 0 to 46°C, preferably at room temperature.

3. Process according to Claim 1 or 2, in which from 0.5 to 5 gram-atoms of sulphur, preferably from 1.5 to 2.5 gram-atoms, are employed per mole of mercaptan.

4. Process according to one of Claims 1 to 3, in which the mercaptan is an alkyl mercaptan and preferably a tertiary alkyl mercaptan.

5. Process according to one of Claims 1 to 4, consisting in introducing sulphur into a mixture of a mercaptan, a base and carbon disulphide.

6. Process according to Claim 5, in which a volume of carbon disulphide ranging from 0.5 to 1.5 times that of the mercaptan used is applied.

7. Process according to Claim 5 or 6, in which the base employed is a metal hydroxide or an amine, preferably a trialkylamine.

8. Process according to one of Claims 5 to 7, in which at least 0.1 millimole of base is employed per mole of mercaptan.